# FASCICULE DE BREVET EUROPEEN

(11) **EP 0 874 810 B1**
(45) Date de publication et mention de la délivrance du brevet: **06.02.2002**
(21) Numéro de dépôt: 96939126.7
(22) Date de dépôt: 15.11.1996
(51) Int. Cl.: C07C 315/06, C07C 317/04

(54) **PROCEDE DE PURIFICATION DU DIMETHYLSULFOXYDE (DMSO)**
VERFAHREN ZUR REINIGUNG VON DIMETHYLSULFOXYD (DMSO)
METHOD FOR PURIFYING DIMETHYL SULPHOXIDE (DMSO)

(30) Priorité: 17.11.1995 FR 9513641
(43) Date de publication de la demande: 04.11.1998
(73) Titulaire: ELF AQUITAINE PRODUCTION, 92400 Courbevoie (FR)
(72) Inventeur: COMMARIEU, Annie, F-92400 Coubevoie (FR); BAZIN, Isabelle, F-64290 Gan (FR)
(74) Mandataire: Leboulenger, Jean
(86) Numéro de dépôt international: FR9601806
(87) Numéro de publication internationale: WO9719057

(56) Documents cités:
- BE-A- 656 879
- FR-A- 2 014 385
- ANAL. CHEM., vol. 40, no. 12, 1968, pages 1769-1773, XP000563242 A.M. PHIPPS: cité dans la demande
- CHIMIE ANALYTIQUE, vol. 53, no. 5, 1971, pages 310-314, XP002004909 T. CHAUDRON ET AL:

## Description

La présente invention concerne un procédé de purification de Diméthylsulfoxyde (DMSO) et le DMSO ainsi purifié.

Le DMSO disponible actuellement sur le marché est un produit ayant déjà une bonne pureté. Ses spécifications commerciales sont généralement :

| | |
|---|---|
| pureté | ≥ 99,7 % par chromatographie |
| acidité | ≤ 0,04 mg KOH/g par potentiométrie |
| point de cristallisation | ≥ 18,2°C |
| aspect visuel | limpide |
| teneur en eau | ≤ 0,15 % en poids par rapport au poids total. |
| couleur (APHA) | ≤ 10 |

La demande de brevet FR 2 014 385 décrit un procédé de préparation de DMSO purifié. Ce procédé met en oeuvre un échangeur d'ions cationiques qui peut être régénéré par un traitement avec des acides. Par contre, le DMSO traité à l'aide de cet échangeur d'ions contient une très forte teneur en eau. De plus, les exemples indiquent l'utilisation d'une résine fortement basique du type Amberlite IR-A 400 ou Merck III, pour des mélanges ternaires Diméthylsulfure/DMSO/acide sulfurique à 10 %, ce qui entraîne peut être la neutralisation de l'acidité mais également l'introduction du contre-cation de la résine basique dans le mélange ainsi traité. En fait, dans ce procédé la purification semble essentiellement apportée par une distillation fractionnée d'une solution aqueuse de DMSO traitée auparavant par un ou plusieurs échangeurs d'ions.

On a effectué maintenant des analyses de traces métalliques sur plusieurs échantillons ( 1 à 6 ) de DMSO commercial, de différentes provenances. Ces analyses sont rapportées dans le tableau I.

Les concentrations en sodium, fer, potassium, calcium, chrome, cuivre, nickel et zinc ont été mesurées par ICP (spectrométrie d'émission atomique-torche à plasma-, appareil Perkin Elmer, modèle Optima 3000) et sont exprimées en p.p.b. (1 p.p.b.= 1 partie en poids par milliard = 1 µg par kg).

La liste des éléments , métalliques, figurant dans le tableau I, n'est pas exhaustive quant aux éléments métalliques présents dans ces échantillons.

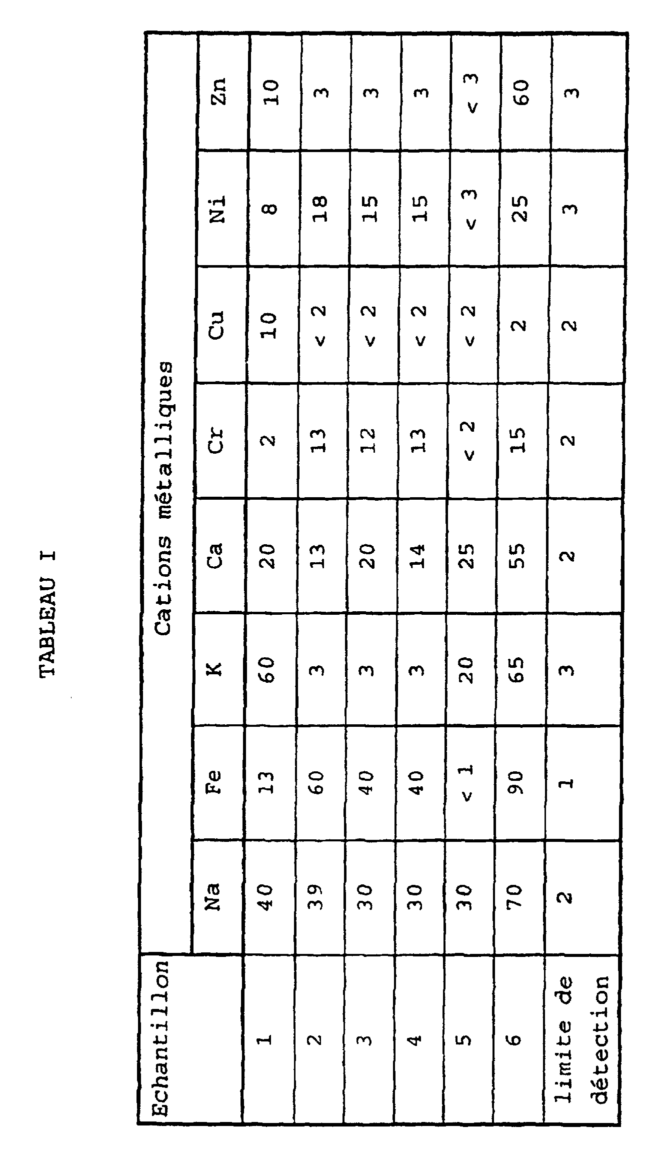

Pour certaines applications, comme par exemple en électronique ou en pharmacie, les DMSO analysés ci-dessus contiennent trop d'impuretés métalliques. En général, un DMSO contenant moins de 10 p.p.b de chaque contaminant métallique, alcalin et alcalino-terreux serait nécessaire pour la plupart des utilisations dans les deux domaines techniques précités.

Le but de la présente invention est de trouver un procédé de purification du DMSO commercial ayant déjà une bonne pureté, mais cependant insuffisante pour certaines applications.

Ce but est atteint par un procédé de purification de Dimethylsulfoxyde (DMSO) pour en diminuer la teneur en cations différents de H⁺, caractérisé en ce qu'il comporte les étapes suivantes :
(a) on sélectionne un DMSO liquide ayant une teneur en eau inférieure ou égale à 0,15 % en poids,
(b) on met ce DMSO en contact avec un solide constitué par une résine échangeuse d'ions de type sulfonique ayant ses groupes actifs sous forme acide sulfonique (SO₃H), cette résine étant à base d'un copolymère polystyrène-divinylbenzène dans lequel le divinylbenzène représente de 50 à 60% en poids du copolymère, et
(c) on sépare ensuite du solide le liquide constitué par du DMSO purifié à de très faibles teneurs en cations différents de H⁺, par tout moyen connu approprié, notamment filtration, percolation ou centrifugation.

L'échange d'ions par la mise en oeuvre de résines est une technique très utilisée pour les milieux aqueux et permet notamment l'obtention d'eau désionisée. L'échange d'anions en milieu DMSO liquide à faible teneur en eau a déjà été réalisé par Alan M. Phipps, Anal. Chem. 40(12) pp. 1769-1773, 1968, dans le but de mesurer les quantités d'anions fixés sur la résine dans des conditions expérimentales s'approchant de l'équilibre thermodynamique.

Nous avons trouvé dans le présente invention que tout cation Mⁿ⁺ ( n nombre entier supérieur ou égal à 1 ) était retenu et échangé par des protons n.H⁺ par une résine sulfonique sous forme protonique, en opérant avec du DMSO à faible teneur en eau ou quasi-anhydre.

Selon l'invention, la résine sulfonique est à base d'un copolymère polystyrène-divinylbenzène. En effet, ces résines ont un squelette résistant aux attaques chimiques et en particulier, elles ne se dissolvent pas dans le DMSO à faible teneur en eau ou quasi-anhydre. Ces résines sont généralement définies par leur taux de divinylbenzène. En effet, ce dernier détermine le taux de réticulation de la résine et donc la taille des pores dans lesquels l'échange cationique se fait à l'échelle atomique.

Selon l'invention, dans le copolymère, le divinylbenzène représente de 50 à 60 % en poids et le polystyrène de 50 à 40 % en poids par rapport au poids total du copolymère, sans tenir compte des groupes sulfonique (SO₃H). Ce taux de divinylbenzène assure une bonne activité cinétique de l'échange des cations Mⁿ⁺ par n.H⁺.

La mise en contact à l'étape (b) a lieu à une température allant de 18,45°C (point de fusion du DMSO) à 120°C. La température de 120°C est la température limite de stabilité thermique des résines.

Avantageusement,la mise en contact du DMSO à l'étape (b) a lieu à une température de 19 à 80°C.

De préférence, cette température va de 20 à 50°C pour le DMSO.

Pour avoir une définition de la qualité du DMSO à faible teneur en eau ou quasi-anhydre susceptible d'être obtenu purifié par le procédé selon l'invention, le fer et le sodium ont été retenus comme éléments traceurs et indicateurs de la teneur générale en cations Mⁿ⁺ notamment métalliques, alcalins et alcalino-terreux.

Le DMSO susceptible d'être obtenu par le procédé selon l'invention se caractérise en ce qu'il comporte une teneur en éléments traceurs cation Fe inférieure ou égale à 1 p.p.b. et cation Na inférieure ou égale à 2 p.p.b., limites respectives de détection de la méthode d'analyse par spectrométrie d'émission atomique-torche à plasma.

L'invention sera mieux comprise à l'aide de la partie expérimentale suivante décrivant des exemples de réalisation de la présente invention.

### Partie expérimentale

### I - Réduction de la teneur en métaux

### I-1. Méthode d'analyse :

La méthode d'analyse de traces métalliques dans le DMSO :
- l'ICP (spectrométrie d'émission atomique-torche à plasma-) : l'échantillon est introduit dans une torche plasma, les différents éléments présents sont excités et émettent des photons dont l'énergie est caractéristique de l'élément puisqu'elle est définie par la structure électronique de l'élément considérée. Nous avons utilisé en routine un appareil Perkin Elmer (modèle Optima 3000).

### I-2. Méthodologie :

Principe : les traces métalliques sont sous forme Mⁿ⁺. Par passage du DMSO sur une résine échangeuse de cations, elle-même sous forme H⁺, on substitue les ions Mⁿ⁺ en solution par nH⁺.

Puisqu'il existe sur le marché de nombreuses résines échangeuses de cations, nous avons choisi de classer les résines suivant leurs performances cinétiques (réactions en batch) et de tester en continu les résines les plus intéressantes. Toutes les résines sont:
- sous forme sulfonique, à base de poly(styrène-divinylbenzène) la teneur initiale, avant réticulation, en divinylbenzène et le fournisseur des résines étant variables,( voir Tableau II ),
   et dans le Tableau. III:
- sous forme sulfonique, à base poly(acrylique-divinylbenzène): C 106,
- sous forme iminodiacétique, à base poly(styrène-divinylbenzène): S 930,
- sous forme aminophosphonique, à base poly(styrène-divinylbenzène): S 940,
- sous forme acétique, à base poly(styrène-divinylbenzène)

### I-3. Sélection des résines :

Principe : par souci de simplifier les analyses, le sodium et/ou le fer ont été choisis comme traceurs représentatifs de l'ensemble des impuretés métalliques contenues dans le DMSO.

Le sodium est caractéristique de la pollution atmosphérique et accidentelle (poussières, environnement) et le fer est caractéristique de la pollution pouvant provenir du process (unité en inox).

### I-4. Réduction de la teneur en cations ( différents de H⁺⁾ dans du DMSO.

### I-4-1. Traitement en batch :

Du DMSO dopé à 1000 p.p.b. de fer et environ 1000 p.p.b. de sodium est mis au contact de résine échangeuse de cations, sous forme H⁺ (2 g de résine pour 100 g de DMSO sous la forme d'un liquide ) à 25°C. Des échantillons de ce liquide sont prélevés au cours du temps. On peut ainsi suivre les évolutions des concentrations en fer et sodium avec le temps.

Toutes les résines sont séchées par mise en suspension dans du méthanol et évaporation sous vide à l'évaporateur rotatif (90°C, 20.10² Pa) jusqu'à observation d'un poids constant.

Lorsque les résines commerciales sont fournies sous forme H⁺, elles sont séchées telles quelles.

Lorsqu'elles sont sous forme Na⁺, elles sont échangées au préalable pour obtenir la forme H⁺ de la manière suivante: 90 ml de résine sont placés dans une colonne. 540 ml d'HCl 5% la traversent à un débit constant et tel que l'opération dure 30 à 45 min. La résine est ensuite rincée à l'eau déionisée jusqu'à neutralité de l'eau sortante.

Dans certains cas, (obtention d'un plateau pour la concentration en sodium, à des teneurs de l'ordre de 200-300 p.p.b., les résines ont été échangées à nouveau par du HCl à 5%.

Le tableau suivant II rassemble pour chaque résine sulfonique à base poly(styrène-divinylbenzène): le nom du fournisseur, la référence commerciale de la résine, la teneur en divinylbenzène et les teneurs en fer et sodium du DMSO en fonction du temps. Les dosages de fer et sodium ont été réalisés par ICP. Lorsque les résultats sont inférieurs à la limite de détection, inhérente à l'appareil, ils sont portés "< limite de détection" (1 p.p.b. pour le fer et 2 p.p.b. pour le sodium).

Le Tableau III rassemble les résultats obtenus avec du DMSO mis en contact avec d'autres résines.

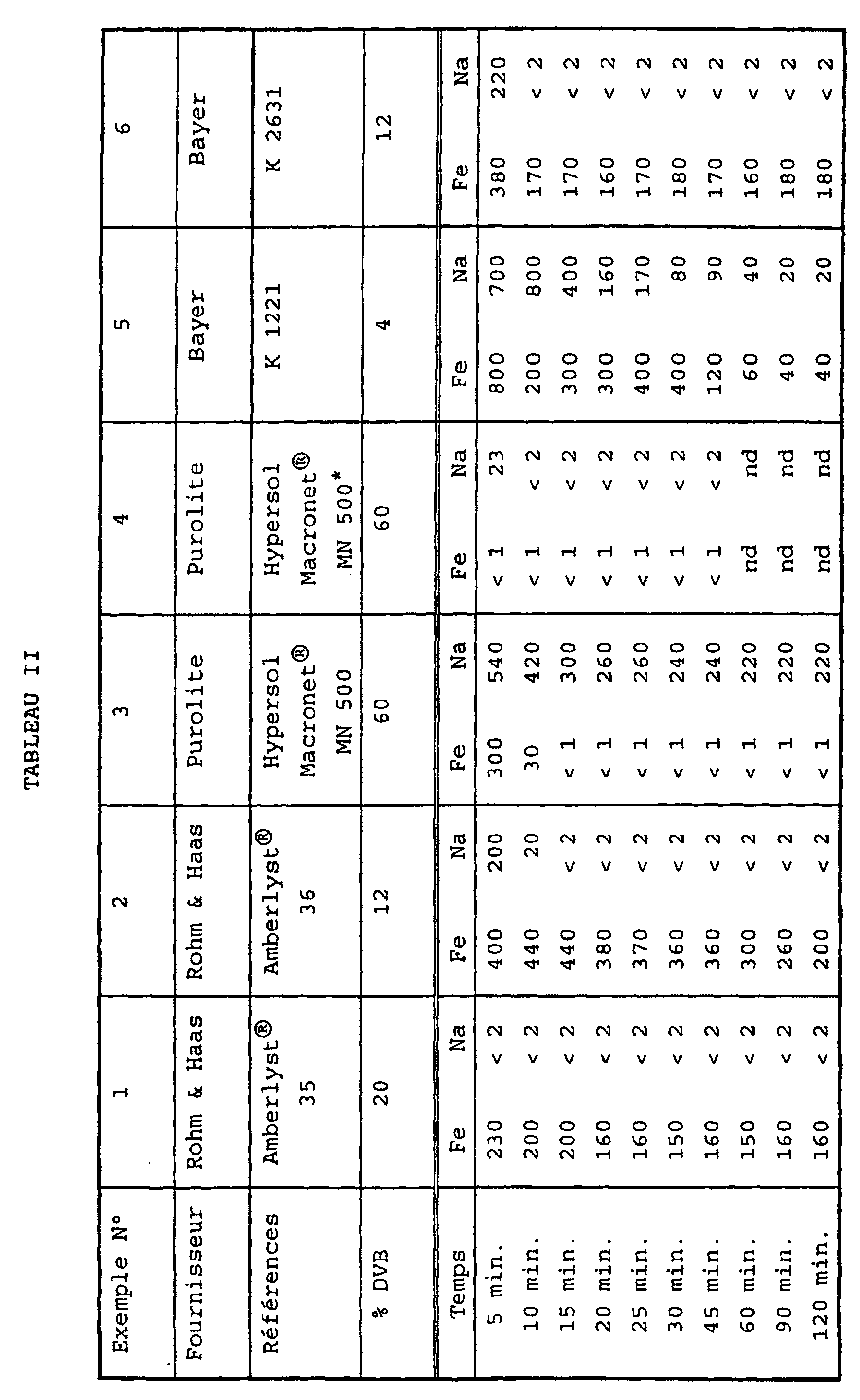

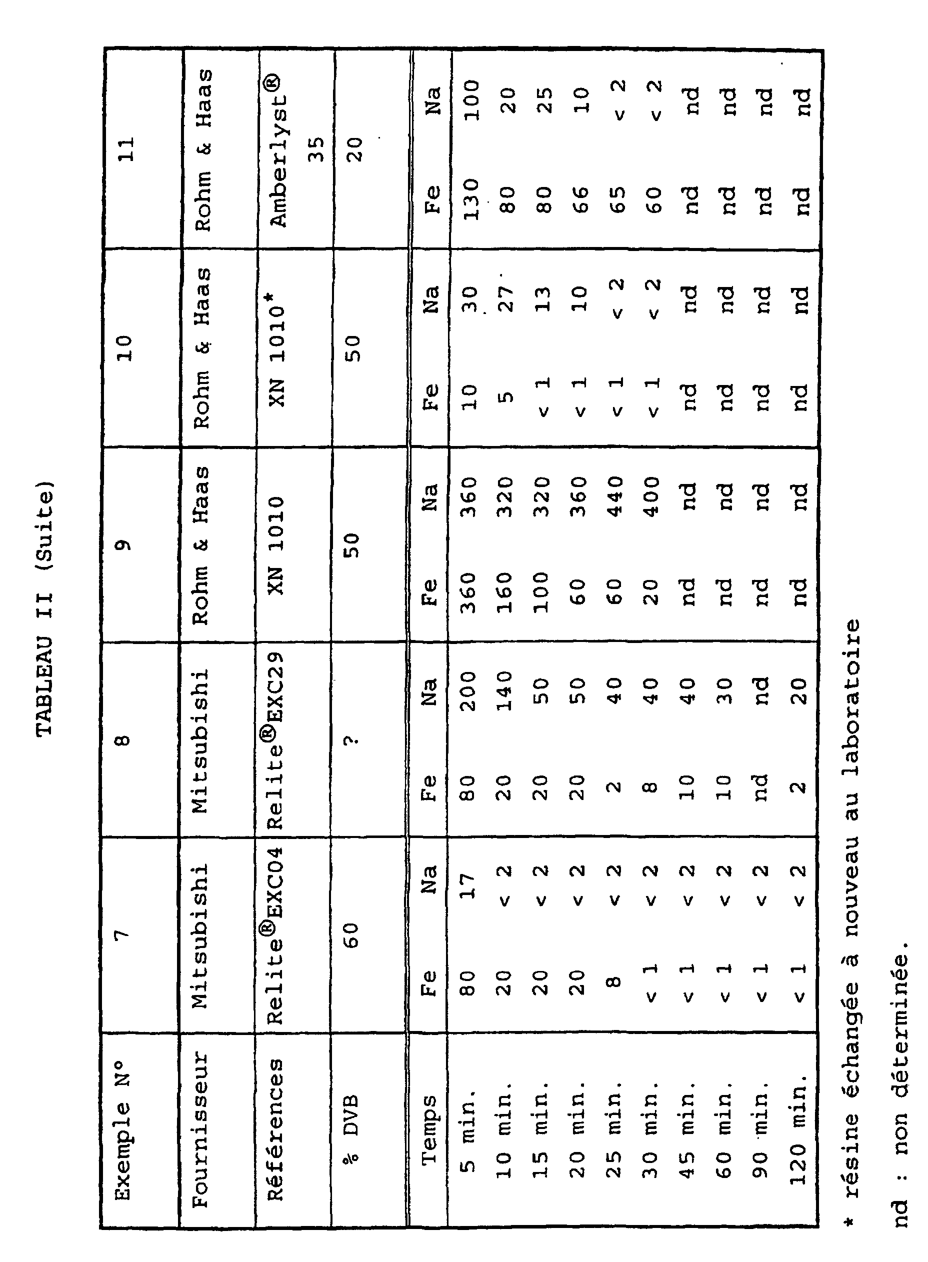

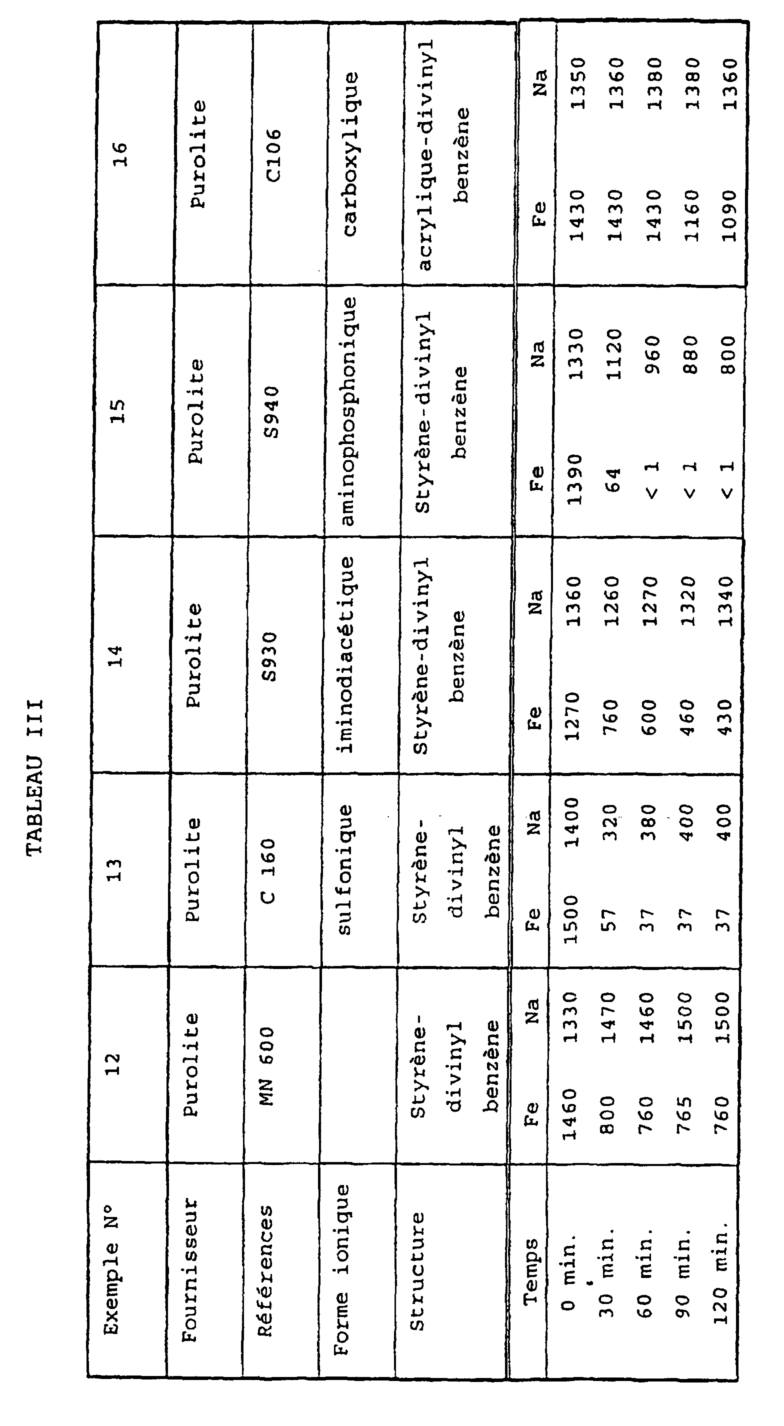

Les résines préférées sont celles qui, dans le test de sélection I-3 ci-dessus, conduisent en 30 min. à des concentrations en fer et en sodium inférieures ou égales aux limites de détection, soit à 1 p.p.b. pour le fer et à 2 p.p.b. pour le sodium.

Toute résine ne figurant pas dans le Tableau II, mais répondant aux critères du test I-3, serait également préférée dans la présente invention.

A l'examen du Tableau II, on voit que les résines Purolite MN 500*, Relite® EXC04, et Rohm & Haas XN 1010* sont les plus actives, et par conséquent préférées. Leur pourcentage en divinylbenzène est de 50 à 60%.

### I-4-2. Traitement en continu :

A partir des résultats en batch, plusieurs tests en continu ont été entrepris.

Conditions de test : selon les règles communément suivies par l'homme de l'art quant aux rapports diamètre de colonne/taille de grain, hauteur de colonne/diamètre de colonne et à la vitesse linéaire pour ne pas avoir de limitation diffusionnelle. La résine est mise en suspension dans 90 ml de DMSO dans un bêcher, légèrement agitée (pour éliminer les bulles d'air) puis cette suspension est introduite dans une colonne en polyéthylène, en position verticale et dont la partie inférieure est équipée d'un fritté en polyéthylène de porosité 70 µm. Le bêcher est rincé avec 10 ml de DMSO. Sous le fritté, la colonne est équipée d'un robinet en Téflon. Ce robinet est fermé au cours de l'opération de remplissage. Une fois la résine déposée et tassée dans la colonne , on ouvre le robinet et alimente la colonne en continu en DMSO grâce à une pompe équipée d'une tête en Téflon. Des prélèvements sont effectués à intervalles réguliers, soit manuellement, soit à l'aide d'un échantillonneur automatique. Tous les tubes et raccords sont en Téflon. Les flacons sont en polyéthylène haute densité.

### I-4-2-1. Exemple 17

Résine Relite® EXC04
Volume de résine sèche : 35 cm³
Taille des grains :0,3-0,8 mm
Diamètre de la colonne : 1.5 cm
Hauteur du lit : 21 cm
Débit de DMSO : 0.35 l/h
Dosage de fer et sodium dans les échantillons par ICP.

Partant d'un DMSO contenant 10 p.p.b. de sodium et 50 p.p.b. de fer, 35 litres de DMSO contenant moins de 2 p.p.b. de sodium et 1 p.p.b. de fer (limites de détection de chaque élément par cette technique) ont été obtenus. L'expérience n'a pas été poursuivie jusqu'à saturation de la résine.

### I-4-2-2. Exemple 18

Résine Relite® EXC29
Volume de résine sèche : 35 cm³
Taille des grains : 0,3-0,8mm
Diamètre de la colonne : 1.5 cm
Hauteur du lit : 26 cm
DMSO initial contenant 40 p.p.b. de sodium et 40 p.p.b. de fer.
Débit de DMSO : 0.40 l/h
Dosage de fer et sodium dans les échantillons par ICP.Voir Tableau IV

**Tableau IV**

| Volume de DMSO traité en litres | Na (p.p.b.) | Fe (p.p.b.) |
|---|---|---|
| 0, (DMSO de départ) | 40 | 40 |
| 1.46 | ≤ 2 | ≤ 1 |
| 4.06 | 25 | ≤ 1 |

### I-4-2-3. Exemple 19

Résine Hypersol Macronet® MN 500
Volume de résine sèche : 35 cm³
Taille des grains : 0,3-1,2 mm
Diamètre de la colonne : 1.5 cm
Hauteur du lit : 21 cm
DMSO initial contenant 25 p.p.b. de sodium et 78 p.p.b. de fer
Débit de DMSO : environ 2 l/h
Dosage de fer et sodium dans les échantillons par ICP.

Les résultats apparaissent dans le Tableau V suivant:

**Tableau V**

| Temps en h | Volume traité en litres | Na en p.p.b. | Fe en p.p.b. |
|---|---|---|---|
| 0 | 0 | 25 | 78 |
| 104 | 208 | ≤ 2 | ≤ 1 |
| 120 | 240 | 20 | ≤ 1 |

### I-4-2-4. Exemple 20

Résine Relite® EXC04
Volume de résine sèche : 32 cm³
Taille des grains : 0,3-0,8 mm
Diamètre de la colonne : 1.5 cm
Hauteur du lit : 19 cm
DMSO initial contenant 20 p.p.b. de sodium et 40 p.p.b. de fer
Débit de DMSO : environ 2 l/h
Dosage de fer et sodium dans les échantillons par ICP.

Les résultats apparaissent dans le Tableau VI suivant:

**Tableau VI**

| Temps en h | Volume traité en litres | Na en p.p.b. | Fe en p.p.b. |
|---|---|---|---|
| 0 | 0 | 20 | 40 |
| 53 | 106 | ≤ 2 | ≤ 1 |
| 77 | 154 | ≤ 2 | ≤ 1 |

### I-4-2-5. Exemples 21 et 22.

Résine Hypersol Macronet® MN 500
Volume de résine sèche: 31 cm³
Taille des grains: 0,3-0,8 mm
Diamètre interne de la colonne: 2 cm
Hauteur du lit: 10 cm
DMSO initial : 10 p.p.b. de sodium et 20 p.p.b. de fer
Débit DMSO: 3,14 l.h⁻¹ ( résultats Tableau VII )
Débit DMSO: 8,3 l.h⁻¹ ( résultats Tableau VIII )

**Tableau VII**

| Volume équivalent 1 de DMSO/l de résine* | Na p.p.b. | Fe p.p.b. |
|---|---|---|
| 0 | < 2 | < 1 |
| 1700 | < 2 | < 1 |
| 2400 | < 2 | < 1 |
| 4100 | < 2 | < 1 |
| 7200 | < 2 | 25 |
| 8948 | < 2 | 26 |

| | | |
|---|---|---|
| * volume de DMSO traité par volume de résine, en litres. | | |

**Tableau VIII**

| Volume équivalent 1 de DMSO/l de résine* | Na p.p.b. | Fe p.p.b. |
|---|---|---|
| 0 | < 2 | < 1 |
| 4887 | < 2 | < 1 |
| 6761 | < 2 | 4 |
| 11313 | < 2 | 5 |
| 13319 | 5 | 5 |
| 17806 | 18 | 24 |
| 19613 | 25 | 25 |

| | | |
|---|---|---|
| * volume de DMSO traité par volume de résine, en litres. Il a été vérifié au cours de ces essais des exemples 21 et 22 que les teneurs en calcium, zinc, magnésium et silicium, étaient respectivement inférieures à 2, 2, 1, et 2 p.p.b. | | |

## Revendications

1. Procédé de purification de Diméthylsulfoxyde (DMSO) pour en diminuer la teneur en cations différents de H⁺, **caractérisé en ce qu'**il comporte les étapes suivantes :
(a) on sélectionne un DMSO liquide ayant une teneur pondérale en eau inférieure ou égale à 0,15 %,
(b) on met ce DMSO en contact avec un solide constitué par une résine échangeuse d'ions de type sulfonique ayant ses groupes actifs sous forme acide sulfonique (SO₃H), cette résine étant à base d'un copolymère polystyrène-divinylbenzène dans lequel le divinylbenzène représente de 50 à 60% en poids du copolymère, et
(c) on sépare ensuite du solide le liquide constitué par du DMSO purifié à très faibles teneurs en cations différents de H⁺, par tout moyen connu approprié, notamment filtration, percolation ou centrifugation.

2. Procédé selon la revendication 1, **caractérisé en ce que** la mise en contact de l'étape (b) a lieu à une température de 19 à 80°C.

3. Procédé selon la revendication 2, **caractérisé en ce que** la température est de 20 à 50°C.

4. DMSO susceptible d'être obtenu par un procédé selon l'une des revendications 1 à 3, **caractérisé en ce qu'**il comporte une teneur en éléments traceurs cation Pe inférieure ou égale à 1 p.p.b. et cation Na inférieure ou égale à 2 p.p.b., limites respectives de détection de la méthode d'analyse par spectrométrie d'émission atomique-torche à plasma.

## Patentansprüche

1. Verfahren zur Reinigung von Dimethylsulfoxid (DMSO) zur Verringerung des Gehaltes an anderen Kationen als H⁺, **dadurch gekennzeichnet, daß** es die folgenden Verfahrensschritte umfaßt:
(a) Man wählt ein flüssiges DMSO mit einem Wassergehalt von weniger oder gleich 0,15 Gew.-% aus;
(b) man bringt dieses DMSO mit einem Feststoff in Kontakt, der aus einem Ionenaustauscherharz vom Sulfonsäuretyp besteht, dessen aktive Gruppen in der Sulfonsäureform (SO₃H) vorliegen, wobei dieses Harz auf Basis eines Polystyrols/Divenylbenzol-Copolymers vorliegt, bei dem das Divenylbenzol 50 bis 60 Gew.-% des Copolymers darstellt; und
(c) anschließend trennt man den Feststoff von der Flüssigkeit, die aus gereinigtem DMSO mit einem sehr geringen Gehalt an von H⁺ verschiedenen Kationen besteht, mittels jedes bekannten, geeigneten Verfahrens ab, insbesondere mittels Filtration, Perkolation oder Zentrifugieren.

2. Verfahren nach Anspruch 1, **dadurch gekennzeichnet, daß** das Inkontaktbringen in Verfahrensschritt (b) bei einer Temperatur von 19 bis 80 °C stattfindet.

3. Verfahren nach Anspruch 2, **dadurch gekennzeichnet, daß** die Temperatur 20 bis 50 °C beträgt.

4. DMSO, erhältlich durch ein Verfahren nach einem der Ansprüche 1 bis 3, **dadurch gekennzeichnet, daß** es einen Gehalt des kationischen Spurenelementes Fe von weniger oder gleich 1 ppb und des kationischen Spurenelementes Na von weniger oder gleich 2 ppb aufweist, was der jeweiligen Nachweisgrenze des Analyseverfahrens mittels Plasmastrahlatomemissionsspektrometrie entspricht.

## Claims

1. Process for the purification of dimethyl sulphoxide (DMSO) in order to decrease the content of cations other than H⁺ therein, **characterized in that** it includes the following steps:
(a) a liquid DMSO having a water content by weight of less than or equal to 0.15% is selected,
(b) this DMSO is placed in contact with a solid consisting of an ion exchange resin of sulphonic type having its active groups in sulphonic acid (SO₃H) form, this resin being based on a polystyrene-divinylbenzene copolymer, in which the divinylbenzene represents from 50 to 60% by weight of the copolymer, and
(c) the liquid consisting of the purified DMSO with very low contents of cations other than H⁺ is then separated from the solid by any suitable known means, in particular filtration, percolation or centrifugation.

2. Process according to Claim 1, **characterized in that** the placing in contact of step (b) takes place at a temperature of from 19 to 80°C.

3. Process according to Claim 2, **characterized in that** the temperature is from 20 to 50°C.

4. DMSO capable of being obtained by a process according to one of Claims 1 to 3, **characterized in that** it has a content of Fe cation tracer elements of less than or equal to 1 ppb and a content of Na cation tracer elements of less than or equal to 2 ppb, the respective detection limits of the method of analysis by plasma-torch atomic emission spectrometry.
